(19) Europäisches Patentamt / European Patent Office / Office européen des brevets

(11) **EP 1 857 099 A1**

(12) **DEMANDE DE BREVET EUROPEEN**

(43) Date de publication:
21.11.2007 Bulletin 2007/47

(51) Int Cl.:
*A61K 8/97* *(2006.01)* *A61Q 19/00* *(2006.01)*
*A61K 36/41* *(2006.01)*

(21) Numéro de dépôt: 07009360.4

(22) Date de dépôt: 10.05.2007

(84) Etats contractants désignés:
AT BE BG CH CY CZ DE DK EE ES FI FR GB GR
HU IE IS IT LI LT LU LV MC MT NL PL PT RO SE
SI SK TR
Etats d'extension désignés:
AL BA HR MK YU

(30) Priorité: 10.05.2006 FR 0604119
19.09.2006 FR 0608191

(71) Demandeur: **Laboratoires Dermatologiques d'Uriage**
**92404 Courbevoie Cedex (FR)**

(72) Inventeur: **Lefeuvre, Luc**
**78580 Bazemont (FR)**

(74) Mandataire: **Burtin, Jean-François**
**Cabinet GEFIB,**
**55 rue Aristide Briand**
**92309 Levallois-Perret Cedex (FR)**

(54) **Compositions cosmétiques ou dermatologiques à base d'extraits de Kalanchoe linearifolia**

(57) La présente invention a pour objet la réalisation de compositions cosmétiques ou dermatologiques contenant, à titre de principe actif, au moins un extrait de *Kalanchoe linearifolia*, et l'utilisation en traitement préventif ou curatif de l'hypersensibilité cutanée et du vieillissement cutané.

EP 1 857 099 A1

**Description**

La présente invention se rapporte au domaine des nécessités de la vie et plus particulièrement au domaine de la cosmétologie et de la dermatologie.

La présente invention se rapporte plus particulièrement à l'utilisation d'extraits de plantes à des fins cosmétiques ou dermatologiques.

La présente invention a spécifiquement pour objet la réalisation de compositions cosmétiques ou dermatologiques contenant, à titre de principe actif, au moins un extrait de *Kalanchoe linearifolia*, en association ou en mélange avec au moins un excipient et/ou véhicule approprié.

*Kalanchoe linearifolia* appartient à la famille des Crassulacées. Le genre Kalanchoe comprend approximativement 120 espèces, localisées majoritairement en Afrique, plus particulièrement à Madagascar. Des extraits de Kalanchoe comme *Kalanchoe brasiliensis* ont déjà montré une activité anti-inflammatoire (Mourão R.H. et. al., Phytotherapy Research 1999, 352-354).

La médecine traditionnelle malgache utilise le jus issu des fleurs fraîches broyées de *Kalanchoe linearifolia* en application topique, notamment pour le traitement de l'eczema.

Bien que certaines espèces apparentées soient connues, aucune littérature spécifique ne décrit les propriétés cosmétiques ou dermatologiques de *Kalanchoe linearifolia*.

Or, la Demanderesse a constaté que des extraits de *Kalanchoe linearifolia* présentent des propriétés intéressantes, notamment en terme d'activité anti-inflammatoire. D'une manière générale, les extraits de *Kalanchoe linearifolia* ont une toxicité très faible ; ils sont aptes à une utilisation cosmétique ou dermatologique.

Les utilisations cosmétiques et dermatologiques considérées pour les compositions contenant ces extraits de plantes concernent notamment le traitement de l'hypersensibilité cutanée et du vieillissement cutané.

Les utilisations cosmétiques comprennent, d'une manière générale et non exhaustive, le traitement préventif et/ou curatif des peaux sensibles et intolérantes, des peaux hyper-réactives, des peaux sujettes aux rougeurs et des peaux grasses à imperfections. Elles comprennent également le traitement préventif et/ou curatif des peaux marquées par les signes du vieillissement, tels que l'amincissement de la peau, les rides et ridules, le flétrissement de la peau ou le manque d'élasticité.

Les utilisations dermatologiques comprennent, de manière non exhaustive, l'usage dans les cas de dermatite atopique (eczema), de psoriasis, de prurit, de rosacée, de dermite séborrhéique et d'acné.

Un extrait de *Kalanchoe linearifolia* utilisé selon l'invention peut être préparé à partir de matériel végétal issu de la plante entière ou de partie de plante comme les feuilles, les tiges, les fleurs, les pétales, les fruits ou les racines.

Selon une forme préférentielle de l'invention, un extrait de *Kalanchoe linearifolia* est préparé à partir de matériel végétal issu des feuilles et des tiges de la plante.

Toute méthode d'extraction connue de l'homme du métier peut être utilisée pour préparer un extrait de *Kadanchoe linearifolia* utilisé selon l'invention.

On peut notamment utiliser les méthodes d'extraction par des solvants comme l'eau, les alcools ou les solvants organiques. On peut également utiliser des mélanges de ces différents solvants, comme par exemple des mélanges hydroalcooliques. Les extractions peuvent notamment avoir lieu à température ambiante ou à une température plus élevée, selon le point d'ébullition du solvant utilisé.

Préférentiellement, les extractions ont lieu à une température comprise entre 20 et 70 °C.

Parmi les alcools pouvant être utilisés comme solvant d'extraction, on peut notamment citer l'éthanol.

Parmi les solvants organiques pouvant être utilisés comme solvant d'extraction, on peut notamment citer l'acétate d'éthyle, le dichlorométhane et les alcanes comme l'hexane, le cyclohexane, l'heptane ou l'octane.

On peut également réaliser une série d'extractions successives par différents solvants, chaque phase d'extraction étant concentrée et le concentrât étant soumis à une nouvelle extraction par un solvant différent. Cette méthode permet de réaliser plusieurs extraits au contenu spécifique, pouvant présenter des propriétés originales.

Par exemple, on peut effectuer une première extraction du matériel végétal à l'aide d'un solvant alcoolique ou hydroalcoolique comme l'éthanol ou un mélange éthanol/eau, concentrer la phase d'extraction obtenue et effectuer une extraction du concentrât à l'aide d'un solvant organique, comme un alcane ou l'acétate d'éthyle. Cette opération peut être répétée plusieurs fois à l'aide de différents solvants.

Selon une forme de l'invention, un extrait de *Kalanchoe linearifolia* constitue le principe actif des compositions selon l'invention.

Selon une autre forme de l'invention, le principe actif des compositions selon l'invention est constitué de l'association d'un extrait de *Kalanchoe linearifolia* avec un ou plusieurs autres composés naturels ou synthétiques, purs ou sous forme d'extraits végétaux, d'action complémentaire, similaire ou synergique.

Selon une forme préférentielle de l'invention, le principe actif des compositions selon l'invention est constitué de l'association d'un extrait de *Kalanchoe linearifolia* avec un extrait de *Sida acuta*. Cette plante tropicale appartient à la famille des Malvacées. Des extraits de cette plante présentent des propriétés anti-inflammatoires et cicatrisantes.

Un extrait de *Sida acuta* utilisé selon l'invention peut être préparé à partir de matériel végétal issu de la plante entière ou de partie de plante comme les feuilles, les tiges, les fleurs, les pétales, les fruits ou les racines.

Selon une forme préférentielle de l'invention, un extrait de *Sida acuta* est préparé à partir de matériel végétal issu des feuilles et des tiges de la plante.

Toute méthode d'extraction connue de l'homme du métier peut être utilisée pour préparer un extrait de *Sida acuta* utilisé selon l'invention, notamment les méthodes décrites ici pour la préparation d'extraits de *Kalanchoe linearifolia*.

Un extrait de de *Kalanchoe linearifolia* ou une composition contenant l'un au moins de ces extraits, sont utilisés selon l'invention en application topique sur la peau et/ou les ongles et/ou les cheveux et/ou les muqueuses externes.

La quantité d'extrait utilisable selon l'invention peut varier dans une large mesure en fonction de l'effet recherché.

Pour donner un ordre de grandeur, on peut utiliser l'extrait à l'état pur en une quantité représentant de 0,0001 à 20% du poids total de la composition, et préférentiellement en une quantité représentant de 0,01 à 10% du poids total de la composition.

Les compositions selon l'invention comprennent un support cosmétiquement ou dermatologiquement acceptable, en association ou en mélange avec le principe actif. Elles peuvent renfermer les adjuvants couramment utilisés dans les domaines cosmétiques et dermatologiques, comme par exemple des agents émollients ou adoucissants, des gélifiants, des antioxydants, des solvants, des filtres, des conservateurs, des pigments ou des parfums. Elles peuvent se présenter sous une forme compatible avec une application topique, comme les solutions aqueuses, hydroalcooliques ou huileuses, les dispersions, les suspensions, les émulsions huile dans eau ou eau dans huile, les gels aqueux ou huileux, les crèmes, les laits, les pommades, les lotions, les pâtes, les poudres, les mousses, les sticks ou les aérosols.

Les propriétés pharmacologiques, notamment anti-inflammatoires des compositions selon l'invention, conduisent à des utilisations cosmétiques telles que le traitement préventif et/ou curatif des troubles de la peau, comme par exemple les peaux sensibles et intolérantes, les peaux hyper-réactives, les peaux sujettes aux rougeurs et/ou les peaux grasses à imperfections, ainsi que le traitement préventif et/ou curatif des peaux marquées par les signes du vieillissement, comme la peau amincie, les rides et ridules, la peau flétrie ou manquant d'élasticité.

Les propriétés notamment anti-inflammatoires des compositions selon l'invention conduisent à des utilisations dermatologiques telles que l'usage dans les cas de dermatite atopique (eczema), de psoriasis, de prurit, de rosacée, de dermite séborrhéique ou d'acné.

Les exemples suivants illustrent l'invention sans toutefois la limiter :

**Exemple 1 : Réalisation d'un extrait de plante utilisé selon l'invention**

Ces méthodes peuvent être appliquées aussi bien à la réalisation d'un extrait de *Kalanchoe linearifolia* qu'à la réalisation d'un extrait de *Sida acuta*. La matière végétale utilisée est issue des feuilles et des tiges de la plante.

**Exemple 1.1 : Réalisation de l'extrait brut (fraction f1) de *Kalanchoe linearifolia***

1000 g de matière végétale séchée sont dispersés dans 5 L d'éthanol à 70°. Le mélange est agité durant 1 heure à 50 °C, puis filtré sur entonnoir, le résidu solide étant rincé par 2 L d'éthanol. La phase alcoolique est concentrée sous pression réduite. Le résidu est repris dans 1,5 litre d'eau et lyophilisé. Un solide brun est obtenu. Dans les exemples suivants, cet extrait sera dénommé « fraction f1 ».

**Exemple 1.2 : Réalisation de l'extrait à l'aide de solvants peu polaires (fraction f2) de *Kalanchoe linearifolia***

La fraction f1 précédemment obtenue est mise en solution dans 500 mL d'éthanol. La solution est extraite par 3 x 200 mL d'hexane. Les phases hexaniques sont rassemblées puis concentrées. Un produit visqueux est obtenu. Dans les exemples suivants, cet extrait sera dénommé « fraction f2 ».

**Exemple 1.3 : Réalisation de l'extrait à l'aide d'un solvant de polarité intermédiaire (fraction f3) de *Kalanchoe linearifolia***

La phase éthanolique de l'étape précédente est concentrée sous pression réduite et reprise dans 500 mL d'eau. Cette solution aqueuse est extraite par 3 * 200 mL d'acétate d'éthyle. Les phases organiques sont rassemblées puis concentrées. Un produit visqueux est obtenu. Dans les exemples suivants, cet extrait sera dénommé « fraction f3 ».

**Exemple 1.4: Réalisation d'un extrait hydrosoluble (fraction f4) de *Kalanchoe linearifolia***

La phase aqueuse de l'étape précédente est lyophilisée. Un solide brun est obtenu. Dans les exemples suivants, cet extrait sera dénommé « fraction f4 ».

**Exemple 1.5 : Réalisation d'un extrait (fraction f5)de *Kalanchoe linearifolia***

100 g de matière végétale séchée sont dispersés dans 2,5 L d'heptane. La suspension est soumise à une extraction au soxhlet à reflux du solvant pendant 4 heures. Après filtration sur entonnoir, la matière végétale est récupérée et ajoutée à 1 L d'éthanol à 70°. Le mélange est agité durant 7 heures au reflux du solvant, puis filtré sur entonnoir. Le résidu solide est ajouté à 1 L d'éthanol à 70° et le mélange est laissé au repos durant 3 jours. Après filtration, les phases alcooliques sont rassemblées et concentrées sous pression réduite. Le résidu est repris dans 1,5 litre d'eau et lyophilisé. Un solide brun est obtenu. La composition de cet extrait est très proche de celle de la fraction f3.

**Exemple 2: Evaluation de l'activité anti-inflammatoire d'extraits de *Kalanchoe linearifolia***

1- OBJECTIFS

Il s'agit de tester les fractions de *Kalanchoe linearifolia* (KLN) dans un test discriminatoire anti-inflammatoire. Les fractions KLN f1, f2, f3, f4 ont été étudiées.

2-CONDITIONS EXPERIMENTALES

L'inflammation est provoquée par injection intra-péritonéale d'une solution d'acide acétique 0,6% qui provoque une inflammation du réseau mésentérique chez la souris Swiss. Cet état inflammatoire se traduit par une augmentation de la perméabilité capillaire de la paroi endothéliale avec pour conséquence un chimiotactisme accru. Le témoin de cette augmentation de la perméabilité, est le Bleu Evans, colorant injecté au préalable par voie intraveineuse dans la veine caudale des souris. Une substance à caractère anti-inflammatoire réduit cette perméabilité et par conséquent l'invasivité par le colorant de la cavité abdominale (mesure par D.O.)

3-METHODOLOGIE

3.1-Plan expérimental

1 dose unique a été testée dans cette étude: 1g/kg (n = 6)
la concentration en Bleu Evans (BE) sera déterminée par une méthode colorimétrique. Le produit de référence utilisé pour évaluer les extraits est la phénylbutazone (PNB) à la dose de 100 mg/kg.

3.2-Critère d'évaluation

La réduction de l'inflammation se traduit par une diminution du passage du colorant (Bleu d'Evans) dans la cavité abdominale donc de sa concentration dans l'exsudat.

3.3-Pertinence de la méthode

Ce test est très utilisé pour évaluer un nouvel anti-inflammatoire en première intention. Il a été décrit par Whittle pour la première fois en 1964 et est encore largement utilisé en screening d'évaluation.

3.4-Déroulement de l'essai

*3.4.1 Race de souris utilisée*

Des souris des deux sexes, de race Swiss pesant entre 27 et 30 g ont été utilisées. Dix huit heures avant l'expérience, les animaux ont été mis à jeun, tout en ayant un accès libre à l'eau.
Les animaux ont été répartis par lot de 6 souris, un lot ayant reçu uniquement de l'eau distillée, a servi de lot témoin.

*3.4.2 Vole d'administration des extraits*

Ces extraits ont été administrés par voie orale, par gavage. Ils ont été dilués dans de l'eau distillée à la dose de 1 g/kg. 4 lots de traités sont ainsi constitués.
La phénylbutazone (PNB) a été utilisée à la dose de 100 mg/kg comme produit de référence.

3.5 -Protocole expérimental

*3.5.1 Perméabilité capillaire*

45 minutes après l'administration des extraits, une solution de colorant (du bleu d'Evans : BE) à la concentration de 0.5% préparée dans une solution physiologique de NaCl (0.9%) a été injectée par voie intraveineuse dans la veine caudale des souris à raison de 10 ml/kg de poids corporel.

15 minutes après l'injection du BE, une solution d'acide acétique (0.6%) a été injectée par voie intra-péritonéale à raison de 10 ml/kg.

Après 30 minutes, les animaux ont été sacrifiés et leur cavité abdominale a été rincée avec 5 ml d'eau distillée contenant 500 UI d'héparine. L'eau de rinçage a été quantitativement collectée et centrifugée à la vitesse de 2000 t/min

La concentration en BE dans le surnageant a été déterminée à l'aide d'un spectrophotomètre à la longueur d'onde de 620 nm (Whittle, 1964).

L'activité inhibitrice des extraits de plantes ainsi que celle de la substance de référence a été exprimée en pourcentage de la perméabilité capillaire chez les animaux traités par rapport au témoin.

*3.5.1 Calcul du pourcentage d'inhibition*

$$\text{Pourcentage d'inhibition} = 1 - (DO_2/DO_1 \times 100)$$

Où:

$DO_1$ = 1 a moyenne de la densité optique du BE chez les animaux témoins
$DO_2$ = 1 a moyenne de la densité optique du BE chez les animaux traités.

Les activités des différents extraits ont été comparées à celle du PNB.

4- RESULTATS ET DISCUSSION : KLN

Le tableau 2 ci-dessous montre les variations individuelles de l'inhibition de la perméabilité capillaire provoquée par l'acide acétique injecté par voie i.p. chez la souris, après administration per os de différentes fractions. Chaque valeur est la moyenne de 6 observations.

*Tableau 2*

| EXTRAIT n = 6 | PNB (0,1 g) | KLN f1 | KLN f2 | KLN f3 | KLN f4 | Excipient témoins |
|---|---|---|---|---|---|---|
| % Inhibition Moyenne ± s.e.m. | 71,9 ±9,3 | 53,9 ±2,9 | 17,7 ±4,7 | 61,3 ±6,3 | 33 ±6,6 | -3,1 ±1,22 |
| *'t'//témoins* | *P<0,01* | *P<0,05* | *NS* | *P<0,01* | *P<0,05* | *NS* |

Les fractions KLN f1 et f3 testées dans cet essai témoignent d'une activité anti-inflammatoire dans le cadre de ce test de screening, à la dose élevée de 1g/kg (extraits bruts non connus quant à leur teneur en actifs).

Dans ce test, le PNB à la dose de 0.1 g/kg provoque une inhibition de l'inflammation à l'acide acétique à la valeur de 71,9% par rapport au groupe d'animaux témoins non traités (eux-mêmes ayant eu une augmentation de leur perméabilité capillaire de 120% par rapport aux animaux normaux après injection d'acide acétique).

L'extrait brut f1 et la fraction f3 sont anti-inflammatoires de façon significative. La modération de la perméabilité cellulaire atteste de l'efficacité de f1 du fait des molécules sélectionnées dans f3.

Les fractions f2 et f4 doivent être considérées comme peu actives dans ce test, bien que f4 soit significativement différent du lot des animaux témoins au stade inflammatoire.

Conclusion:

L'activité anti-inflammatoire de KLN est confirmée, surtout en ce qui concerne la fraction f3.

**Exemple 3 : Composition cosmétiques et dermatologiques contenant des extraits de *Kalanchoe linearifolia***

Réalisation d'un onguent :

| Dénomination INCI | Concentration (%) |
|---|---|
| Petrolatum | 25 - 75 |
| Paraffinum liquidum | 15 - 30 |
| Polydecene | 5 - 20 |
| Dipentaerythrityl pentahydroxystearate / isostearate | 1 - 3 |
| Glycerin | 1 - 10 |
| Extrait de plante | 0,1 - 5 |
| *Kalanchoe linearifolia* | 0,1 - 5 |
| TOTAL | Qsp 100 |

Réalisation d'une émulsion :

| Dénomination INCI | Concentration (%) |
|---|---|
| Caprylic/capric triglyceride | 5 - 15 |
| Stearalkonium hectorite / propylene carbonate | 1 - 10 |
| Myristyl myristate | 1 - 5 |
| Hexyl decyl stearate | 5 - 15 |
| Isostearyl diglyceryl succinate | 1 - 10 |
| Extrait de plante | 0,1 - 5 |
| *Kalanchoe linearifolia* | 0,1 - 5 |
| Aqua | Qsp 100 |

Les extraits de plantes utilisés ici sont les fractions f5.

**Revendications**

1. Compositions cosmétiques ou dermatologiques destinées à l'application topique, **caractérisées en ce qu'**elles renferment, à titre de principe actif, au moins un extrait de *Kalanchoe linearifolia,* en association ou en mélange avec un excipient ou véhicule approprié.

2. Compositions cosmétiques ou dermatologiques selon la revendication 1, **caractérisées en ce qu'**elles renferment en outre un extrait de *Sida acuta*.

3. Compositions cosmétiques ou dermatologiques selon la revendication 1 ou la revendication 2, **caractérisées en ce que** l'extrait ou les extraits sont obtenus selon un procédé impliquant une étape d'extraction de la plante ou d'une partie de la plante séchée, à l'aide d'un solvant alcoolique ou hydroalcoolique.

4. Compositions cosmétiques ou dermatologiques selon l'une des revendications précédentes, **caractérisées en ce que** la quantité de l'extrait ou de chacun des différents extraits est comprise entre 0,0001 et 20% du poids total de la composition.

5. Compositions cosmétiques ou dermatologiques selon l'une des revendications précédentes, **caractérisées en ce que** la quantité de l'extrait ou de chacun des différents extraits est comprise entre 0,01 et 10% du poids total de la composition.

6. Compositions cosmétiques ou dermatologiques selon l'une des revendications précédentes, **caractérisées en ce qu'**elles renferment en outre un ou plusieurs principes actifs complémentaires.

# EP 1 857 099 A1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | EP 1 291 011 A1 (USPENSKAYA SVETLANA IGOREVNA [RU]) 12 mars 2003 (2003-03-12) * page 2, alinéa 2; exemples 2,3,8 * * page 3, alinéa 12 * * page 6, alinéa 35 - alinéa 36 * | 1-6 | INV. A61K8/97 A61Q19/00 A61K36/41 |
| Y | EP 1 352 640 A (YAKULT HONSHA KK [JP]) 15 octobre 2003 (2003-10-15) * revendications 1,2; exemples 2,3,8 * * page 4, alinéa 18 - alinéa 19 * * page 9, alinéa 45; tableaux 2,3 * * page 12, alinéa 47 * | 1-6 | |
| Y | TADEG H ET AL: "Antimicrobial activities of some selected traditional Ethiopian medicinal plants used in the treatment of skin disorders" JOURNAL OF ETHNOPHARMACOLOGY, ELSEVIER SCIENTIFIC PUBLISHERS LTD, IE, vol. 100, no. 1-2, 22 août 2005 (2005-08-22), pages 168-175, XP004996523 ISSN: 0378-8741 * tableaux 1-3 * Results and discussion | 1,3-6 | |
| Y | MOTHANA R A A ET AL: "Antimicrobial activity of some medicinal plants of the island Soqotra" JOURNAL OF ETHNOPHARMACOLOGY, ELSEVIER SCIENTIFIC PUBLISHERS LTD, IE, vol. 96, no. 1-2, 4 janvier 2005 (2005-01-04), pages 177-181, XP004657439 ISSN: 0378-8741 Results and discussion* tableaux 1,2 * -/-- | 1,3-6 | **DOMAINES TECHNIQUES RECHERCHES (IPC)** A61K A61Q |

Le présent rapport a été établi pour toutes les revendications

| Lieu de la recherche | Date d'achèvement de la recherche | Examinateur |
|---|---|---|
| Munich | 23 juillet 2007 | Loloiu, Teodora |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**Office européen des brevets**

**RAPPORT DE RECHERCHE EUROPEENNE**

Numéro de la demande

EP 07 00 9360

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | "Kalanchoe pinnata"<br>TROPICAL PLANT DATABASE, [Online]<br>3 décembre 2005 (2005-12-03), XP002432102<br>Extrait de l'Internet:<br>URL:http://web.archive.org/web/20051203084<br>720/http://www.rain-tree.com/coirama.htm><br>[extrait le 2007-04-30]<br>* le document en entier *<br>----- | 1,3-6 | |
| Y | RAINTREE NUTRITION: "Ethnomedical information on Coirama (Kalanchoe pinnata)"[Online]<br>21 mai 2005 (2005-05-21), XP002432164<br>Extrait de l'Internet:<br>URL:http://web.archive.org/web/20050521062<br>139/http://www.rain-tree.com/kalanchoe-tra<br>ditional-uses.pdf> [extrait le 2007-05-04]<br>* le document en entier *<br>----- | 1,3-6 | |
| Y | PURKAYASTHA J ET AL: "Ethnobotany of medicinal plants from Dibru-Saikhowa Biosphere Reserve of Northeast India"<br>FITOTERAPIA, IDB HOLDING, MILAN, IT,<br>vol. 76, no. 1, janvier 2005 (2005-01),<br>pages 121-127, XP004722492<br>ISSN: 0367-326X<br>* page 126; tableau 1 *<br>----- | 2 | DOMAINES TECHNIQUES RECHERCHES (IPC) |
| P,A | NGUELEFACK ET AL: "Analgesic and anticonvulsant effects of extracts from the leaves of Kalanchoe crenata (Andrews) Haworth (Crassulaceae)"<br>JOURNAL OF ETHNOPHARMACOLOGY, ELSEVIER SCIENTIFIC PUBLISHERS LTD, IE,<br>vol. 106, no. 1,<br>15 juin 2006 (2006-06-15), pages 70-75,<br>XP005435657<br>ISSN: 0378-8741<br>* le document en entier *<br>----- | 1-6 | |

Le présent rapport a été établi pour toutes les revendications

| Lieu de la recherche | Date d'achèvement de la recherche | Examinateur |
|---|---|---|
| Munich | 23 juillet 2007 | Loloiu, Teodora |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**EP 1 857 099 A1**

## ANNEXE AU RAPPORT DE RECHERCHE EUROPEENNE
## RELATIF A LA DEMANDE DE BREVET EUROPEEN NO.

EP 07 00 9360

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

23-07-2007

| Document brevet cité au rapport de recherche | | Date de publication | Membre(s) de la famille de brevet(s) | | Date de publication |
|---|---|---|---|---|---|
| EP 1291011 | A1 | 12-03-2003 | WO | 0195873 A1 | 20-12-2001 |
| | | | RU | 2161950 C1 | 20-01-2001 |
| EP 1352640 | A | 15-10-2003 | BR | 0116098 A | 30-12-2003 |
| | | | WO | 0247656 A1 | 20-06-2002 |
| | | | JP | 2002179581 A | 26-06-2002 |
| | | | US | 2004028643 A1 | 12-02-2004 |

EPO FORM P0460

Pour tout renseignement concernant cette annexe : voir Journal Officiel de l'Office européen des brevets, No.12/82

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **MOURÃO R.H.** *Phytotherapy Research,* 1999, 352-354